# EUROPÄISCHE PATENTANMELDUNG

(11) **EP 1 199 751 A2**
(43) Veröffentlichungstag der Anmeldung: **24.04.2002**
(21) Anmeldenummer: 01126917.2
(22) Anmeldetag: 23.06.1999
(51) Int. Cl.: H01L 25/065, H01L 25/07, G01N 27/403, G01N 27/414, G01N 27/28, G01N 33/487

(54) **Chip-Anordnung**

(30) Priorität: 30.06.1998 DE 19829121
(62) Teilanmeldung aus: 99112071.8
(71) Anmelder: Micronas GmbH, 79008 Freiburg (DE)
(72) Erfinder: Igel, Günter, Dipl.-Ing., 79331 Teningen (DE); Lehmann, Mirko, Dipl.-Phys., 79102 Freiburg (DE); Sieben, Ulrich, Dr., 79276 Reute (DE); Gahle, Hans-Jürgen, Dr., 79312 Emmendingen (DE); Baumann, Werner, Dr., 77815 Bühl (DE); Ehret, Ralf, Dr., 18057 Rostock (DE)
(74) Vertreter: Bickel, Michael

(57) **Zusammenfassung**

Eine Chip-Anordnung (1) hat eine Substratplatte (2), die zwei Durchbrüche (3) mit darin eingesetzten Trägerchips (4) aufweist. Diese haben jeweils an einer Trägerchip-Oberfläche wenigstens eine den Durchbruch (3) durchsetzende integrierte Leiterbahn (7), die ein elektrisches oder elektronisches Bauelement (5) einem elektrischen Anschlußkontakt (8) verbindet. Das Bauelement (5) des einen Trägerchips (4) ist als Strahlungs-Emitter und das Bauelement (5) des anderen Trägerchips (4) als Empfänger ausgebildet. Zwischen dem Strahlungs-Emitter und dem Empfänger ist eine Meßstrecke angeordnet. Die Trägerchips (4) sind derart in den Durchbruch (3) eingesetzt, daß sie mit ihren Enden die einander abgewandten flachseitigen Oberflächen (9, 9') der Substratplatte (2) überragen und dadurch Überstände (10, 10') bilden. An dem die eine Oberfläche (9) überragenden Überstand (10) ist jeweils das Bauelement und an dem die andere Oberfläche (9') überragenden Überstand (10') der Anschlußkontakt (8) angeordnet. Zwischen der Substratplatte (2) und dem Trägerchip (4) eine Abdichtung vorgesehen.

## Beschreibung

Die Erfindung betrifft eine Chip-Anordnung mit einer Substratplatte, die wenigstens einen Durchbruch aufweist, in den ein Trägerchip eingesetzt oder einsetzbar ist, der an einer Trägerchip-Oberfläche wenigstens eine integrierte Leiterbahn aufweist, die wenigstens ein elektrisches oder elektronisches Bauelement mit zumindest einem elektrischen Anschlußkontakt verbindet, wobei der Trägerchip derart in den Durchbruch eingesetzt oder einsetzbar ist, daß er mit seinen Enden die einander abgewandten flachseitigen Oberflächen der Substratplatte überragt und dadurch Überstände bildet, wobei an dem die eine Oberfläche überragenden Überstand das Bauelement und an dem die andere Oberfläche überragenden Überstand der Anschlußkontakt angeordnet ist, wobei die das Bauelement und den Anschlußkontakt miteinander verbindende Leiterbahn den Durchbruch der Substratplatte durchsetzt, und wobei zwischen der Substratplatte und dem Trägerchip eine Abdichtung vorgesehen ist.

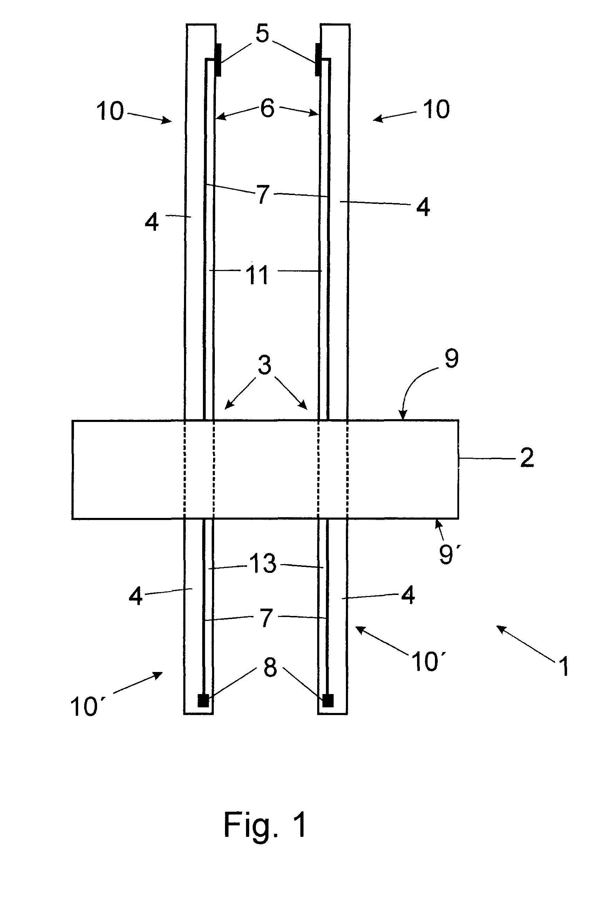

Eine derartige Chip-Anordnung ist aus WO 87 05747 A, Fig. 4 bekannt. Sie weist einen Trägerchip mit zwei ionensensitiven Sensoren auf, die über Leiterbahnen mit Anschlußkontakten des Trägerchips verbunden sind. Der Trägerchip ist so in die Öffnung einer Wandung eingesetzt, daß er mit seinen Enden die einander abgewandten Seiten der Wandung überragt und dadurch Überstände bildet. An dem einen Überstand sind die Sensoren und an dem anderen Überstand die Anschlußkontakte angeordnet. Die Leiterbahnen, welche die Sensoren mit den Anschlußkontakten verbinden, sind durch die Öffnung der Wandung hindurchgeführt. Diese Chip-Anordnung ermöglicht zwar die Messung von lonenkonzentrationen, nicht jedoch eine Streulicht- oder Durchlichtmessung.

Aus WO 87 05747 A, Fig. 2 ist außerdem eine gattungsfremde Chip-Anordnung bekannt, die eine Substratplatte mit einem Durchbruch aufweist, in den ein Trägerchip derart eingesetzt ist, daß er mit seinen Enden die einander abgewandten flachseitigen Oberflächen der Substratplatte überragt und dadurch Überstände bildet. Der Trägerchip weist einen etwa U-förmigen optischen Wellenleiter auf, der mit seinen beiden U-Schenkeln jeweils den Durchbruch der Substratplatte durchsetzt, so daß die freien Enden der U-Schenkel an dem einen Überstand und der U-Quersteg des Wellenleiters an dem anderen Überstand angeordnet sind. Das freie Ende des einen U-Schenkels ist einem optischen Strahlungs-Emitter und das des anderen U-Schenkels mit einem optischen Empfänger zugewandt. Letzterer ist mit elektrischen Leiterbahnen des Trägerchips verbunden. An dem den U-Quersteg aufweisenden Überstand ist eine optische Zelle in den Lichtleiter geschaltet, die aus einem chemosensitiven Material besteht, das seine optischen Eigenschaften verändert und somit das in dem Wellenleiter geführte Licht moduliert, wenn es einem Medium mit bestimmten chemischen Eigenschaften in Kontakt kommt. Auch diese Chip-Anordnung ermöglicht keine Streulicht- oder Durchlichtmessung.

Es besteht deshalb die Aufgabe, eine Chip-Anordnung der eingangs genannten Art zu schaffen, die bei einem einfachen Aufbau eine Streulicht- oder Durchlichtmessung eines Mediums ermöglicht, wobei die elektrischen Anschlußkontakte des Trägerchips vor Korrosion durch das Medium geschützt sein sollen.

Die Lösung dieser Aufgabe besteht bei einer Anordnung der eingangs genannten Art darin, daß in die Substratplatte wenigstens zwei Trägerchips eingesetzt sind, daß einer der Trägerchips zumindest ein als Strahlungs-Emitter ausgebildetes Bauelement und der andere Trägerchip zumindest ein als Empfänger ausgebildetes, dem Strahlungs-Emitter zugeordnetes Bauelement hat und daß zwischen dem Strahlungs-Emitter und dem Empfänger eine Meßstrecke angeordnet ist.

Mit einer solchen Chip-Anordnung kann eine Streulicht- oder Durchlichtmessung durchgeführt werden. Dabei können die beiden Trägerchips gegebenenfalls gleichzeitig auch einen mechanischen Filter bilden, so daß nur Partikel bis zu einer bestimmten, durch den Abstand der Trägerchips vorgegebenen Größe in die Meßstrecke gelangen können. Das elektrische oder elektronische Bauelement ist an dem an der einen Substratplatten-Oberfläche befindlichen Überstand und der damit über die in dem Trägerchip integrierte Leiterbahn verbundene Anschlußkontakt an dem an der anderen Substratplatten-Oberfläche befindlichen Überstand angeordnet. Der Anschlußkontakt befindet sich also an der dem elektrischen oder elektronischen Bauelement abgewandten Rückseite der Substratplatte, so daß die im Bereich des das Bauelement aufweisenden Überstands befindlichen Leiterbahnbereiche vollständig mit einer Passivierungsschicht abgedeckt werden können. Eine solche Passivierungsschicht kann beispielsweise in Dünnschichttechnologie mit großer Genauigkeit und Feuchtigkeitsfestigkeit hergestellt werden, so daß eine Korrosion an der in den Trägerchip integrierten Leiterbahn durch das mit dem elektrischen oder elektronischen Bauelement zu untersuchende oder zu behandelnde Medium weitestgehend vermieden wird. Die zwischen dem Trägerchip und der Substratplatte angeordnete Abdichtung verhindert, daß das an der Vorderseite der Substratplatte befindliche Medium zu dem an der Rückseite der Substratplatte angeordneten Anschlußkontakt gelangen kann. Der in der Substratplatte angeordnete Durchbruch kann beispielsweise mittels Ultraschallbohren in die Substratplatte eingebracht werden. Die Chip-Anordnung ist somit einfach und kostengünstig herstellbar. Da eine Kunststoffmasse zum Eingießen von Bondpads entfallen kann, weist die Chip-Anordnung außerdem besonders kompakte Abmessungen auf.

Für eine Untersuchung oder Behandlung von biologischen Zellen ist es vorteilhaft, wenn der Abstand zwischen dem elektrischen oder elektronischen Bauelement des einen Trägerchips und dem anderen Trägerchip an den Durchmesser einer biologischen Zelle angepaßt ist und vorzugsweise größer als 4 µm und kleiner als 55 µm ist. Dadurch kann sich eine Zelle zwischen dem das elektrische oder elektronische Bauelement aufweisenden Überstand des einen Trägerchips und dem Überstand des anderen Trägerchips unmittelbar an dem Bauelement anlagern, während Partikel, deren Abmessungen größer sind als der Zelldurchmesser von dem Bauelement ferngehalten werden.

Vorteilhaft ist, wenn die das elektrische oder elektronische Bauelement aufweisende Trägerchip-Oberfläche des einen Trägerchips und die dieser zugewandte Oberfläche des anderen Trägerchips trichterförmig schräg zueinander verlaufen. Dadurch ergibt sich ein trichterförmiger Kanal, der für ein an der Substratplatte befindliches Medium einen strömungsrichtungsabhängigen Filter bildet.

Zweckmäßigerweise ist wenigstens ein Trägerchip lösbar mit der Substratplatte verbindbar. Der Trägerchip kann dann gegebenenfalls leicht ausgetauscht werden, wenn das Bauelement seine vorgesehene Lebensdauer erreicht hat oder wenn es durch einen Kontakt mit einem zu untersuchenden oder zu behandelnden, chemisch agressiven Medium, einmal ausfallen sollte.

Bei einer bevorzugten und besonders vorteilhaften Ausführungsform der Erfindung ist wenigstens ein Trägerchip mit einem den Durchbruch begrenzenden Wandungsbereich der Substratplatte verklebt. Der zwischen dem Trägerchip und der Substratplatte angeordnete Klebstoff dient dann einerseits dazu, den Trägerchip an der Substratplatte zu fixieren und dichtet andererseits aber auch den Durchbruch der Substratplatte gegen den Trägerchip ab, so daß ein an der Vorderseite der Substratplatte im Bereich des elektrischen oder elektronischen Bauelements befindliches Medium nicht an die den Anschlußkontakt aufweisende Rückseite der Substratplatte gelangen kann. Der Klebstoff gleicht außerdem Toleranzen in den Abmessungen des Trägerchips und/oder dem in der Substratplatte befindlichen Wandungsdurchbruch, in den der Trägerchip eingesetzt ist, aus. Die Chip-Anordnung ist dadurch noch einfacher und kostengünstiger herstellbar.

Zweckmäßigerweise ist wenigstens ein Trägerchip mit seiner Erstreckungsebene rechtwinklig zu einer flachseitigen Oberfläche der Substratplatte angeordnet. Die den Durchbruch begrenzenden Seitenflächen der Substratplatte können dann rechtwinklig zu deren flachseitiger Oberfläche angeordnet sein, was das Einbringen des Durchbruchs in die Substratplatte erleichtert.

Eine besonders vorteilhafte Ausführungsform der Erfindung sieht vor, daß die das elektrische oder elektronische Bauelement aufweisende Trägerchip-Oberfläche wenigstens eines Trägerchips schräg zur flachseitigen Oberfläche der Substratplatte angeordnet ist und mit dieser vorzugsweise einen spitzen Winkel einschließt. Das elektrische oder elektronische Bauelement ist dann in einem durch den Trägerchip und die Substratplatte begrenzten Eckbereich angeordnet, so daß nur Partikel, die eine durch die Abmessungen des Eckbereichs vorgegebene Größe nicht überschreiten, mit dem elektrischen oder elektronischen Bauelement in Kontakt geraten können. Somit ergibt sich ein einfach aufgebauter mechanischer Filter, der das Vordringen größerer Partikel zu dem Bauelement verhindert.

Eine vorteilhafte Ausführungsform der Erfindung sieht vor, daß wenigstens ein Trägerchip bei der Montage der Chip-Anordnung in wenigstens zwei unterschiedlichen Lagen in den Durchbruch der Substratplatte einsetzbar ist, daß in einer dieser Lagen wenigstens ein elektrisches oder elektronisches Bauelement an einem eine flachseitige Oberfläche der Substratplatte überragenden Überstand des Trägerchips und der (die) diesem (diesen) Bauelement(en) zugeordnete(n) Anschlußkontakt(e) an dem die andere flachseitige Oberfläche der Substratplatte überragenden Überstand angeordnet ist, und daß in der anderen Lage des Trägerchips das (die) Bauelement(e) und der (die) Anschlußkontakt(e) an demselben, eine flachseitige Oberfläche der Substratplatte überragenden Überstand des Trägerchips angeordnet sind. Dadurch ist es möglich, das Baulelement durch entsprechendes Einsetzen des Trägerchips in die Substratplatte nur für die Dauer einer Messung oder einer Behandlung mit einem an einer flachseitigen Oberflächen der Substratplatte befindlichen Objekt, beispielsweise einem chemisch agressiven Medium, in Berührung zu bringen, während das Baulelement außerhalb der Meßoder Behandlungsphase an der dem Objekt abgewandten flachseitigen Oberflächen der Substratplatte angeordnet ist. Das Bauelement kommt also nur vorübergehend mit dem agressiven Medium in Verbindung, wodurch sich seine Lebensdauer entsprechend verlängert.

Bei einer besonders vorteilhaften Weiterbildung der Erfindung ist vorgesehen, daß wenigstens ein Trägerchip bei der Montage der Chip-Anordnung in wenigstens zwei unterschiedlichen Lagen in den Durchbruch der Substratplatte einsetzbar ist, daß der Trägerchip wenigstens zwei elektrische oder elektronische Bauelemente aufweist, die jeweils mittels wenigstens einer Leiterbahn mit zumindest einem ihnen jeweils zugeordneten elektrischen Anschlußkontakt verbunden sind, und daß je nach gewählter Lage des Trägerchips jeweils wenigstens eines dieser Bauelemente an einem eine flachseitige Oberfläche der Substratplatte überragenden Überstand des Trägerchips und der (die) diesem (diesen) Bauelement(en) zugeordnete(n) Anschlußkontakt an dem die andere flachseitige Oberfläche der Substratplatte überragenden Überstand angeordnet ist. Dadurch ist je nach gewählter Lage des Trägerchips ein anderes Bauelement oder sogar mehrere andere Bauelemente an dem in Gebrauchsstellung dem zu untersuchenden oder zu behandelnden Objekt zugewandten Überstand des Trägerchips angeordnet. Bei einem Trägerchip mit mehreren gleichen Bauelementen verlängert sich dadurch die Lebensdauer der Chip-Anordnung entsprechend, da ein Bauelement, das beispielsweise durch einen längeren Kontakt mit einem chemisch agressiven Medium unbrauchbar geworden ist, durch entsprechendes Umsetzen des Trägerchips auf einfache Weise durch ein anderes, funktionsfähiges Bauelement ersetzt werden kann. Der Trägerchip kann aber auch voneinander verschiedene Bauelemente aufweisen. Dadurch ergibt sich ein Bausatz zum Erstellen einer Chip-Anordnung, mit dem je nach gewählter Lage des Trägerchips in dem Durchbruch der Substratplatte unterschiedliche Chip-Anordnungen hergestellt werden können. Die elektrischen oder elektronischen Bauelemente können beispielsweise am Umfang des Trägerchips verteilt in dessen flachseitige Oberfläche integriert sein, wobei der Trägerchip in unterschiedlichen Drehlagen in Bezug zu der Normalen auf diese Oberfläche in die Substratplatte einsetzbar ist. Abhängig von der jeweiligen Drehlage des Trägerchips sind dann jeweils andere Bauelemente an der Vorderseite der Substratplatte angeordnet, während die diesen zugeordneten Anschlußkontakte sich jeweils an der Rückseite der Substratplatte befinden.

Zum Filtern kleiner Partikel, beispielsweise solcher mit einem Durchmesser, der kleiner als 1 µm ist, ist es vorteilhaft, wenn die Trägerchips derart aneinander anliegen, daß das elektrische oder elektronische Bauelement eines ersten Trägerchips durch einen zweiten Trägerchip überdeckt ist, daß als Abstandshalter an dem ersten Trägerchip mindestens ein seitlich über die Oberflächenebene des Bauelements vorstehender, an dem zweiten Trägerchip anliegender Bereich und/oder an dem zweiten Trägerchip ein seitlich über den das Bauelement überdeckenden Oberflächenbereich vorstehender, an dem ersten Trägerchip anliegender Bereich angeordnet ist, derart, daß zwischen dem Bauelement und dem zweiten Trägerchip ein einen Zugang zu dem Bauelement bildender Freiraum oder Spalt angeordnet ist. Dadurch ist der zweite Trägerchip bei der Montage der Chip-Anordnung auf einfache Weise und mit großer Genauigkeit an dem ersten Trägerchip positionierbar. Der gegenüber dem Bauelement vorstehende Bereich kann mit bekannten Verfahren der Halbleitertechnik, beispielsweise in Maskentechnik mit großer Maßgenauigkeit hergestellt werden, was insbesondere die Realisierung kleiner Spaltmaße bzw. Freiräume zwischen dem Bauelement und dem zweiten Trägerchip mit eng tolerierten Abmessungen ermöglicht. Der an dem vorstehende Trägerchip-Bereich anliegende zweite Trägerchip kann einen im wesentlichen ebenen, dem Bauelement zugewandten, parallel zu dessen Oberflächenebene angeordneten und vorzugsweise an dem vorstehenden Trägerchip-Bereich anliegenden Oberflächenbereich aufweisen. Der erste Trägerchip kann plan an dem zweiten Trägerchip anliegen. Die Herstellung des gegenüber dem Bauelement vorstehenden Trägerchip-Bereichs kann beispielsweise in der Weise erfolgen, daß in die Oberfläche des ersten Trägerchips eine Vertiefung eingeätzt wird, in welcher das Bauelement angeordnet wird oder daß an bestimmten Stellen der Oberfläche des ersten Trägerchips wenigstens eine Schicht aufgedampft oder aufgetragen wird.

Vorteilhaft ist, wenn die Substratplatte aus einem elastischen Material besteht. Die Substratplatte kann dann in Erstreckungsrichtung mit einer Zug- oder Druckkraft beaufschlagt werden. Dadurch kann die Filtercharakteristik des mechanischen Filters auf einfache Weise an die Größe der zu untersuchenden oder zu behandelnden Partikel angepaßt werden. Gegebenenfalls kann die Substratplatte auch als biegbare Folie ausgebildet sein. Die Chip-Anordnung ist dann noch besser handhabbar.

Die Chip-Anordnung kann noch kostengünstiger hergestellt werden, wenn die Substratplatte wenigstens vier in einer Ebene angeordnete Plattenteile aufweist, wenn zueinander benachbarte Plattenteile jeweils an ihren einander zugewandten Randbereichen vorzugsweise durch eine Klebung miteinander verbunden sind, und wenn der Durchbruch durch einen zwischen den Plattenteilen befindlichen Freiraum gebildet ist. Dadurch kann ein teueres Bohren des Durchbruchs, beispielsweise mittels Ultraschall oder eines Laserstrahls entfallen. Auch kann an dem den Durchbruch begrenzenden Rand der Substratplatte ein Grat, wie er beispielsweise beim Laserbohren auftreten kann, vermieden werden. Die einzelnen Plattenteile weisen vorzugsweise jeweils gerade Ränder auf und können beispielsweise durch Trennschleifen oder Sägen zugeschnitten werden.

Besonders vorteilhaft ist, wenn wenigstens zwei erste Plattenteile jeweils zumindest einen geraden Randbereich aufweisen, mit denen sie parallel zueinander und einander zugewandt angeordnet sind, und wenn zwischen den ersten Plattenteilen in Erstreckungsrichtung der geraden Randbereiche durch den Durchbruch voneinander beabstandet zumindest zwei zweite Plattenteile angeordnet sind, die jeweils an ihren parallel zueinander verlaufenden Rändern mit den geraden Randbereichen der ersten Plattenteile insbesondere durch eine Klebung verbunden sind. Die aneinander anliegenden ersten und zweiten Plattenteile können dann vor dem Anbringen der Klebung in Richtung ihrer geraden Randbereiche gegeneinander verschoben werden, wodurch die Länge des in der Substratplatte befindlichen Durchbruchs auf einfache Weise verändert und an die Abmessungen des darin einzusetzenden Trägerchips angepasst werden kann.

Zweckmäßigerweise ist der quer zur Trägerchip-Erstreckungsebene angeordnete stirnseitige Endbereich des Trägerchips zumindest im Bereich des das Bauelement aufweisenden Überstands mit einer Isolationsschicht abgedeckt. Dadurch wird bei einem Halbleiter-Trägerchip ein Kurzschluß zwischen dem Substrat des Trägerchips und einem in den Trägerchip integrierten elektronischen Bauelement vermieden, wenn der das elektronische Bauelement aufweisende Überstand des Trägerchips mit einem elektrisch leitfähigen Medium, zum Beispiel einem Nährmedium für biologische Zellen, in Verbindung gebracht wird.

An der den Anschlußkontakten zugewandten Rückseite der Substratplatte kann eine Leiterplatte angeordnet sein, die mit den Anschlußkontakten verbundene oder verbindbare Anschlussstellen aufweist. Dadurch ergibt sich ein besonders kompakter Aufbau. Die Leiterplatte kann beispielsweise eine Auswertevorrichtung und/oder eine Steuereinrichtung und/oder eine Stromversorgung für die Chip-Anordnung aufweisen. Diese ist an der Rückseite der Substratplatte vor Berührung mit einem zu untersuchenden Medium geschützt.

Nachfolgend ist ein Ausführungsbeispiel der Erfindung anhand der Zeichnung näher erläutert. Die einzige Figur zeigt eine Chip-Anordnung, mit einer Durchbrüche aufweisenden Substratplatte, in die zwei Trägerchips eingesetzt sind, von denen eines einen optischen Sender und das andere einen diesem zugeordneten Empfänger aufweist.

Eine im ganzen mit 1 bezeichnete Chip-Anordnung weist eine Substratplatte 2 auf, die Durchbrüche 3 für zwei einander zugeordnete Trägerchips 4 aufweist. Die Substratplatte 2 kann beispielsweise aus Glas oder einem Halbleitermaterial bestehen.

Einer der Trägerchips 4 weist als Bauelement 5 einen optischen Sender und der andere Trägerchip 4 einen optischen Empfänger auf. Zwischen dem optischen Sender und dem Empfänger ist eine Meßstrecke gebildet. Die Chip-Anordnung kann beispielsweise zur Durchlicht- oder Streulichtmessung und/oder als Lichtschranke verwendet werden.

Die Bauelemente 5 sind an einer flachseitigen Oberfläche 6 des Trägerchips 4 mit Methoden der Halbleitertechnik in den Trägerchip 4 integriert. Die einzelnen Bauelemente 5 sind jeweils mit einer an der Oberfläche 6 des Trägerchips 4 oder im wesentlichen parallel dazu verlaufenden Leiterbahn 7 mit einem Anschlußkontakt 8 verbunden, an dem eine Auswerte- und Steuereinrichtung anschließbar ist.

Die Trägerchips 4 sind so in die Durchbrüche 3 der Substratplatte 2 eingesetzt, daß die Trägerchips 4 jeweils mit ihren Enden die einander abgewandten flachseitigen Oberflächen 9, 9' der Substratplatte 2 überragen und dadurch Überstände 10, 10' bilden, die an den flachseitigen Oberflächen 9, 9' der Substratplatte 2 vorstehen. Dabei sind die Bauelemente 5 an dem einen Überstand 10 und die diesen jeweils zugeordneten elektrischen Anschlußkontakte 8 an dem anderen Überstand 10' angeordnet. Die Leiterbahnen 7 durchsetzen jeweils einen Durchbruch 3 der Substratplatte 2.

Die Trägerchips 4 sind jeweils mit dem den ihnen jeweils zugeordneten Durchbruch 3 der Substratplatte 2 begrenzenden Rand der Substratplatte 2 verklebt, wobei der zwischen diesem Rand und dem Trägerchip 4 befindliche Klebstoff den Trägerchip 4 gegen die Substratplatte 2 abdichtet. Somit sind die an der Rückseite der Substratplatte 2 befindlichen Anschlußkontakte 8 gut gegen ein an der den Bauelementen 5 zugewandten Vorderseite der Substratplatte befindliches, mit den Bauelementen 5 zu untersuchendes Medium, das beispielsweise ein Nährmedium mit darin befindlichen biologischen Zellen sein kann, abgeschirmt. Eine Korrosion an den Anschlußkontakten 8 durch in dem Nährmedium enthaltene Bestandteile, wie beispielsweise Salze oder Ionen, wird dadurch zuverlässig vermieden. Da die Leiterbahnen 7 den Durchbruch 3 der Substratplatte 2 durchsetzen, brauchen bei der Herstellung der Chip-Anordnung zum Verbinden der Bauelemente 5 mit den elektrischen Anschlußkontakten 8 keine Durchkontaktierungen in die Substratplatte 2 eingebracht werden. Die Chip-Anordnung 1 ist dadurch einfach und kostengünstig herstellbar.

Die Trägerchips 4 können jeweils als rechteckiges Plättchen ausgebildet sein, das mit seiner Chipebene und seinen quer dazu verlaufenden Schmalseitenflächen jeweils rechtwinklig zu den flachseitigen Oberflächen 9, 9' der Substratplatte 2 angeordnet ist. Die die Durchbrüche 3 begrenzenden Oberflächen der Substratplatte 2 sind jeweils rechtwinklig zu ihren flachseitigen Oberflächen 9, 9' angeordnet. Dadurch können die Durchbrüche 3 bei der Herstellung der Chip-Anordnung leichter in die Substratplatte 2 eingebracht werden.

Die Leiterbahnen 7 sind mit einer elektrisch isolierenden Dünnfilm-Passivierungsschicht 11 abgedeckt, die beispielsweise aus Siliziumoxid bestehen kann. Durch die Passivierungsschicht 11 sind die Leiterbahnen 7 gegen das Nährmedium elektrisch gut isoliert. Außerdem wird durch die Passivierungsschicht 11 eine Korrosion an den Leiterbahnen 7 durch in dem Nährmedium enthaltene Salze oder Ionen verhindert.

Erwähnt werden soll noch, daß der Trägerchip 4 auch ein Bauelement 5 aufweisen kann, das ein elektrisches Feld aussendet, mit dem eine an der Substratplatte 2 befindliche Zelle beeinflußt oder stimuliert werden kann.

## Patentansprüche

1. Chip-Anordnung (1) mit einer Substratplatte (2), die wenigstens einen Durchbruch (3) aufweist, in den ein Trägerchip (4) eingesetzt oder einsetzbar ist, der an einer Trägerchip-Oberfläche wenigstens eine integrierte Leiterbahn (7) aufweist, die wenigstens ein elektrisches oder elektronisches Bauelement (5) mit zumindest einem elektrischen Anschlußkontakt (8) verbindet, wobei der Trägerchip (4) derart in den Durchbruch (3) eingesetzt oder einsetzbar ist, daß er mit seinen Enden die einander abgewandten flachseitigen Oberflächen (9, 9') der Substratplatte (2) überragt und dadurch Überstände (10, 10') bildet, wobei an dem die eine Oberfläche (9) überragenden Überstand (10) das Bauelement und an dem die andere Oberfläche (9') überragenden Überstand (10') der Anschlußkontakt (8) angeordnet ist, wobei die das Bauelement (5) und den Anschlußkontakt (8) miteinander verbindende Leiterbahn (7) den Durchbruch (3) der Substratplatte (2) durchsetzt, und wobei zwischen der Substratplatte (2) und dem Trägerchip (4) eine Abdichtung vorgesehen ist, **dadurch gekennzeichnet, daß** in die Substratplatte (2) wenigstens zwei Trägerchips (4) eingesetzt sind, daß einer der Trägerchips (4) zumindest ein als Strahlungs-Emitter ausgebildetes Bauelement (5) und der andere Trägerchip (4) zumindest ein als Empfänger ausgebildetes, dem Strahlungs-Emitter zugeordnetes Bauelement (5) hat, und daß zwischen dem Strahlungs-Emitter und dem Empfänger eine Meßstrecke angeordnet ist.

2. Chip-Anordnung (1) nach Anspruch 1, **dadurch gekennzeichnet, daß** der Abstand zwischen dem elektrischen oder elektronischen Bauelement (5) des einen Trägerchips (4) und dem anderen Trägerchip (4) an den Durchmesser einer biologischen Zelle angepaßt ist und vorzugsweise größer als 4 µm und kleiner als 55 µm ist.

3. Chip-Anordnung (1) nach Anspruch 1 oder 2, **dadurch gekennzeichnet, daß** die das elektrische oder elektronische Bauelement (5) aufweisende Trägerchip-Oberfläche (6) des einen Trägerchips (4) und die dieser zugewandte Oberfläche (6) des anderen Trägerchips (4) trichterförmig schräg zueinander verlaufen.

4. Chip-Anordnung (1) nach einem der Ansprüche 1 bis 3, **dadurch gekennzeichnet, daß** die Leiterbahn(en) (7) zumindest im Bereich eines ein elektrisches oder elektronisches Bauelement (5) aufweisenden Überstandes (10) mit einer elektrisch isolierenden Dünnfilm-Passivierungsschicht abgedeckt ist (sind).

5. Chip-Anordnung (1) nach einem der Ansprüche 1 bis 4, **dadurch gekennzeichnet, daß** sich der Querschnitt der die elektrischen und/oder elektronischen Bauelemente (5) aufweisenden Überstände (10) jeweils ausgehend von der Oberfläche (9) der Substratplatte (2) zu der am weitesten vorstehenden Stelle des betreffenden Überstandes (10) verjüngt.

6. Chip-Anordnung (1) nach einem der Ansprüche 1 bis 5, **dadurch gekennzeichnet, daß** wenigstens ein Trägerchip (4) bei der Montage der Chip-Anordnung (1) in wenigstens zwei unterschiedlichen Lagen in den Durchbruch (3) der Substratplatte (2) einsetzbar ist, daß in einer dieser Lagen wenigstens ein elektrisches oder elektronisches Bauelement (5) an einem eine flachseitige Oberfläche der Substratplatte (9) überragenden Überstand (10) des Trägerchips (4) und der (die) diesem (diesen) Bauelement(en) (5) zugeordnete(n) Anschlußkontakt(e) (8) an dem die andere flachseitige Oberfläche (9') der Substratplatte überragenden Überstand (10') angeordnet ist, und daß in der anderen Lage des Trägerchips (4) das (die) Bauelement(e) (5) und der (die) Anschlußkontakt(e) (8) an demselben, eine flachseitige Oberfläche der Substratplatte (9, 9') überragenden Überstand (10, 10') des Trägerchips (4) angeordnet sind.

7. Chip-Anordnung (1) nach einem der Ansprüche 1 bis 6, **dadurch gekennzeichnet, daß** wenigstens ein Trägerchip (4) bei der Montage der Chip-Anordnung (1) in wenigstens zwei unterschiedlichen Lagen in den Durchbruch (3) der Substratplatte (2) einsetzbar ist, daß der Trägerchip (4) wenigstens zwei elektrische oder elektronische Bauelemente (5) aufweist, die jeweils mittels wenigstens einer Leiterbahn (7) mit zumindest einem ihnen jeweils zugeordneten elektrischen Anschlußkontakt (8) verbunden sind, und daß je nach gewählter Lage des Trägerchips (4) jeweils wenigstens eines dieser Bauelemente (5) an einem eine flachseitige Oberfläche der Substratplatte (9) überragenden Überstand (10) des Trägerchips (4) und der (die) diesem (diesen) Bauelement(en) (5) zugeordnete(n) Anschlußkontakt(e) (8) an dem die andere flachseitige Oberfläche (9') der Substratplatte überragenden Überstand (10') angeordnet ist.

8. Chip-Anordnung (1) nach einem der Ansprüche 1 bis 7, **dadurch gekennzeichnet, daß** die Trägerchips (4) derart aneinander anliegen, daß das elektrische oder elektronische Bauelement (5) eines ersten Trägerchips (4) durch einen zweiten Trägerchip (4) überdeckt ist, daß als Abstandshalter an dem ersten Trägerchip (4) mindestens ein seitlich über die Oberflächenebene des Bauelements (5) vorstehender, an dem zweiten Trägerchip (4) anliegender Bereich und/oder an dem zweiten Trägerchip (4) ein seitlich über den das Bauelement (5) überdeckenden Oberflächenbereich vorstehender, an dem ersten Trägerchip (4) anliegender Bereich angeordnet ist, derart, daß zwischen dem Bauelement (5) und dem zweiten Trägerchip (4) ein einen Zugang zu dem Bauelement bildender Freiraum oder Spalt angeordnet ist.

9. Chip-Anordnung (1) nach einem der Ansprüche 1 bis 8, **dadurch gekennzeichnet, daß** die Substratplatte (2) wenigstens vier in einer Ebene angeordnete Plattenteile aufweist, daß zueinander benachbarte Plattenteile jeweils an ihren einander zugewandten Randbereichen vorzugsweise durch eine Klebung miteinander verbunden sind, und daß der Durchbruch durch einen zwischen den Plattenteilen befindlichen Freiraum gebildet ist.

10. Chip-Anordnung (1) nach einem der Ansprüche 1 bis 9, **dadurch gekennzeichnet, daß** wenigstens ein elektrisches oder elektronisches Bauelement (5) mit einer in den Trägerchip (4) integrierten Auswerte- und oder Steuereinheit verbunden ist.
